# EUROPEAN PATENT APPLICATION

(11) **EP 4 246 392 A1**
(43) Date of publication of application: **20.09.2023**
(21) Application number: 22162137.8
(22) Date of filing: 15.03.2022
(51) Int. Cl.: G06Q 10/04, G06Q 10/06, G16H 10/40

(54) **FORECASTING FUTURE LABORATORY PERFORMANCE**

(71) Applicant: F. Hoffmann-La Roche AG, 4070 Basel (CH); Roche Diagnostics GmbH, 68305 Mannheim (DE)
(72) Inventor: ASQUER, Giulia, 6343 Rotkreuz (CH); JANNER-SCHUBINGER, Gabriele Piero, 6343 Rotkreuz (CH); OOSTERBROEK, Edwin, 6343 Rotkreuz (CH); SCHWEIGHAUSER, Stephan, 8153 Ruemlang (CH); STEINERT, Chris, 6343 Rotkreuz (CH); ZEDER, Michael, 6343 Rotkreuz (CH)
(74) Representative: Herren, Barbara

(57) **Abstract**

A computer-implemented method of forecasting future laboratory performance of a laboratory system is presented. The laboratory system comprises a plurality of laboratory instruments configured to perform tests on laboratory test samples, a laboratory middleware, a control unit, and a dashboard display communicatively connected via a network communication connection. The method comprises providing laboratory operator preferences and laboratory constraints from a laboratory operator to an optimization module of the control unit, providing laboratory input data and order data to the optimization module of the control unit, optimizing laboratory configuration based on the laboratory operator preferences, laboratory constraints, laboratory inputs, and order data at the optimization module of the control unit, simulating future laboratory performance of the laboratory system by a simulation module of the control unit based on the optimized laboratory configuration provided by the optimization module and real-time laboratory inputs and order data, and displaying the simulated future laboratory performance and actual laboratory performance on the dashboard display to the laboratory operator.

## Description

### Technical Field

The present disclosure generally relates to a method of predicting, or forecasting, performance of a laboratory system in order to optimize the performance of the laboratory system.

### Background

Typical laboratory performance dashboards only indicate the current and past performance of the laboratory as well as any potential gaps with required performance. Additionally, typical dashboards do not indicate future performance or realistically optimal performance nor do dashboards predict upcoming laboratory events.

Further, current laboratory performance dashboards cannot provide to laboratory operators accurate predictions of when laboratory test results will become available to the laboratory operator. Generally, only rough estimates of laboratory test results availability is currently provided. These rough estimates, typically, rely on simple algorithms that use the analyzer data provided to the laboratory middleware.

Further, currently, there is no means for a laboratory operator to quickly and accurately determine the consequences in terms of laboratory performance due to daily variations of laboratory workloads or when the laboratory operator alters workflow variables, maintenance schedules, and/or laboratory configurations. Instead, when the laboratory does not perform as expected due to the consequences of the alternations from the origin design, the laboratory operator, typically, will complain of longer working days and/or the late delivery of test results to the customers of the laboratory. Therefore, if a dashboard can quickly and accurately demonstrate the effect of such alterations on laboratory performance to the laboratory operator before the alterations are made, time, costs, and resources can be saved for the laboratory.

Document EP 2602625 B1 discloses a method for monitoring a diagnostic test process by simulating the process, receiving data representing the actual progress of the diagnostic test process; and displaying the simulated and actual diagnostic test process.

Document US 7,960,178 discloses a method for processing samples that provides alternatives scheduling processes to an operator in order to accomplish certain tasks.

Document US 9,194,876 discloses a method for making and displaying estimations of when a sample result might be available based on various laboratory data sources.

Document US 9,466,040 discloses a method for predicting congestion in an automatic analysis system and adjusting the timing and taking-out of specimens in order to avoid the congestion.

### Summary

It is an object of the present disclosure to predict, or forecast, performance of a laboratory system in order to optimize the performance of the laboratory system.

According to one aspect of the present disclosure, a computer-implemented method of forecasting future laboratory performance of a laboratory system is disclosed. The laboratory system can comprise a plurality of laboratory instruments configured to perform tests on laboratory test samples, pre-analytical instruments, post-analytical instruments, a laboratory middleware, a control unit, a laboratory test sample transportation system, and a dashboard display communicatively connected via a network communication connection. The method can comprise providing laboratory operator preferences and laboratory constraints from a laboratory operator to an optimization module of the control unit, providing laboratory input data and order data to the optimization module of the control unit, optimizing laboratory configuration based on the laboratory operator preferences, laboratory constraints, laboratory inputs, and order data at the optimization module of the control unit, simulating future laboratory performance of the laboratory system by a simulation module of the control unit based on the optimized laboratory configuration provided by the optimization module and real-time laboratory inputs and order data, and displaying the simulated future laboratory performance and actual laboratory performance on the dashboard display to the laboratory operator.

The laboratory input data and order data can be continuously provided by the laboratory middleware in real-time. Sample collection data and laboratory test sample transportation data can also be continuously provided by the laboratory middleware in real-time.

The real-time laboratory data comprises the status of the resources in the laboratory such as, for example, the status of the laboratory instruments, status of the transportation system, availability of laboratory operators, availability of reagents, availability of consumables, the status of the laboratory test samples, the timing for masking of laboratory instruments, i.e., the ability of the laboratory instruments to accept laboratory test samples, reagent pack assignments and placements, sample loading scheduling, workflow rules and/or combinations thereof.

The status of the laboratory test samples can include, for example, location of the laboratory test samples at any given time, i.e., in which laboratory instrument is the laboratory test sample or at which location in the laboratory, how much processing has occurred and how much remains, i.e., which test operations have been carried out and which test operations still need to occur, how much test sample volume remains, and/or combinations thereof.

Workflow rules can include the rules that can define, for example, at which laboratory instrument do the tests need to be performed on the laboratory test sample and in which order, whether an aliquot needs to be created, and/or combinations thereof.

The simulated future laboratory performance can comprise predicting arrival of test results from the plurality of laboratory instruments.

The displayed simulated future performance can comprise simulated throughputs, turn-around time (TAT) of samples, times to results, buffer levels, sample traffic intensities, instrument loads and workload of laboratory operators, idle times, number of samples, reagents, or tests exceeding a performance acceptance criteria, point-to-point travel times, buffer wait times, walk-away times, number of laboratory operator interactions per time, number of laboratory operators needed, power consumption, water consumption, operational costs, or combinations thereof.

The displayed simulated future performance can comprise indicators of the benefits of the simulated laboratory configuration compared to the current laboratory configuration.

The computer-implemented method can further comprise changing the laboratory configuration of the current laboratory via input from the laboratory operator based on the simulated future laboratory performance.

The computer-implemented method can further comprise triggering an alert when the simulated future laboratory performance and actual laboratory performance deviate from an acceptable level and/or when the simulated test samples arrivals and the actual test samples arrivals deviate from an acceptable level and/or an abnormality is detected.

The computer-implemented method can further comprise indicating on the dashboard display a potential source of the deviation or abnormality.

The computer-implemented method can further comprise calculating estimated future performance based on the optimized laboratory configuration.

The computer-implemented method can further comprise calculating sample loading effects on the simulated future laboratory performance.

The computer-implemented method can further comprise scheduling manual interactions with the laboratory system based on the optimized laboratory configuration.

In some embodiments, a laboratory process may be unknown to the simulation model. In this embodiment, "unknown" can mean that there is no, or not a sufficiently good, model currently available for representing the behavior of this laboratory instrument or manual process, for example. Unknown elements and poor models can lead to unknown or poorly predicted times in the simulation model. However, as data from the corresponding laboratory process is received by the laboratory system, it can be used to improve the simulation model to increase the accuracy of future simulations. As more data is received, the better the model can become in forecasting future events.

The computer-implemented method can further comprise displaying on the dashboard predicted upcoming events with an indication when these predicted upcoming events are expected to occur. Predicted upcoming events can comprises, for example, status changes for the laboratory test samples, laboratory data, or laboratory equipment, publication of test results, the status change of one or multiple sample from being processed to being ready, a sample being ready to be removed from a laboratory instrument, a rack or tray of tests samples being ready to be picked up by the laboratory operator, the load change of a laboratory instrument or change of the number of test samples in test sample queues at laboratory instruments, times for carrying out quality control (QC) or refilling a laboratory instrument with reagents or consumables and the like.

Additional predicted upcoming events can be estimations of when refills will be needed and/or estimations of the start time of laboratory maintenance events and/or estimations of the duration of the laboratory maintenance events, predicting when future laboratory maintenance events may occur, when a complete batch of laboratory test samples has been analyzed, when laboratory test samples are ready to be taken out of a laboratory instrument or laboratory system, times when all or specific test results are published of specific laboratory test samples (e.g. urgent / emergency) such that a validation needs to be done or the test results can be communicated to the medical professionals and the like. Predictions can also help to define when the urgent laboratory tests are completed, such that the laboratory personnel can plan activities that may cause longer TAT times, such as, taking a coffee break, running QC tests, carrying out activities such that manually loading of samples, reagents or consumables may be delayed and the like.

According to a second aspect of the present disclosure, a computer-implemented method of forecasting future laboratory performance of a laboratory system is disclosed. The laboratory system can comprise a plurality of laboratory instruments configured to perform tests on laboratory test samples, a laboratory middleware, a control unit, and a dashboard display communicatively connected via a network communication connection. The method can comprise providing different configurations of the laboratory system from a laboratory operator to a simulation module of the control unit, continuously providing real-time laboratory input data from the plurality of laboratory instruments to the laboratory middleware, continuously providing the real-time laboratory input data and order data from the laboratory middleware to the simulation module, simulating future laboratory performance of the different configurations of the laboratory system by a simulation module of the control unit based on the real-time laboratory inputs and order data, and displaying the simulated future laboratory performances of the different configurations of the laboratory system and actual laboratory performance on the dashboard display to the laboratory operator.

The computer-implemented method can further comprise selecting by the laboratory operator one of the different configurations of the laboratory system based on the simulated future laboratory performances of the different configurations and reconfiguring the laboratory system based on the selected configuration.

The reconfiguring of the laboratory system can be performed manually and/or can be performed automatically.

The computer-implemented method can further comprise optimizing future laboratory performances of the different configurations based on laboratory operator preferences, laboratory constraints, real-time laboratory inputs, and order data at an optimization module.

According to a third aspect of the present disclosure, a laboratory system for forecasting future laboratory performance is disclosed. The laboratory system can comprise a plurality of laboratory instruments configured to perform tests on laboratory test samples, a laboratory middleware communicatively connected to the plurality of laboratory instruments, a dashboard display configured to display performance information of the laboratory system, and a control unit comprising an optimization module and a simulation module and connected to the plurality of laboratory instruments and the laboratory middleware via the network communication connection. The control unit can be configured to provide laboratory operator preferences and laboratory constraints from a laboratory operator to the optimization module and to continuously provide real-time laboratory input data from the plurality of laboratory instruments to the laboratory middleware. The control unit can also be configured to continuously provide the real-time laboratory input data and order data from the laboratory middleware to the optimization module and to optimizing laboratory configuration based on the laboratory operator preferences, laboratory constraints, real-time laboratory inputs, and order data at the optimization module. The control unit can also be configured to simulate future laboratory performance of the laboratory system by the simulation module based on the optimized laboratory configuration provided by the optimization module and real-time laboratory inputs and order data and to display the simulated future laboratory performance and actual laboratory performance on the dashboard display to the laboratory operator.

By forecasting, or predicting, future laboratory performance through the use of simulation, several advantages emerge. Firstly, the time at which a deviation happens can be identified more precisely. For example, normally, a laboratory operator would know a deviation happened between, for example, the real time-to-result and the wanted time-to-result only after the sample test results are published, which would be too late to fix the deviation for that particular sample and, thus, would also cause other samples in the process to be delayed. However, by forecasting future laboratory performance, the workflow step(s) where the deviation may occur such as, for example, at a sample transport step or in the laboratory instrument, can be identified during the test sample process time via comparison of each reported event with the corresponding simulation events and corrected in the real laboratory setting as soon as a deviation is detected.

Additionally, the laboratory system element(s) responsible for the deviation can be identified or, at least, potential laboratory elements possibly responsible for the deviation can be narrowed for determination during the simulation process.

### Brief Description of the Several Views of the Drawings

The following detailed description of specific embodiments of the present disclosure can be best understood when read in conjunction with the following drawings, where like structure is indicated with like reference numerals and in which:
Fig. 1 illustrates a flow chart of the laboratory configuration optimizer and simulation during the laboratory design phase according to an embodiment of the present disclosure.
Fig. 2 illustrates an exemplary user interface dashboard display during the laboratory design phase according to an embodiment of the present disclosure.
Fig. 3 illustrates a flow chart of the run-time application of laboratory configuration with laboratory performance simulation according to an embodiment of the present disclosure.
Fig. 4 illustrates an exemplary actual laboratory performance dashboard display illustrating a histogram of time to results according to an embodiment of the present disclosure.
Fig. 5 illustrates a flow chart of the run-time application of laboratory configuration with laboratory performance simulation with laboratory user input according to an embodiment of the present disclosure.
Fig. 6 illustrates an exemplary user interface dashboard display illustrating a laboratory operator manually changing the laboratory configuration according to an embodiment of the present disclosure.
Fig. 7 illustrates a flow chart of the use of a simulator to check time for performing a laboratory service and/or laboratory maintenance according to an embodiment of the present disclosure.
Fig. 8 illustrates an exemplary user interface dashboard display illustrating the impact of servicing instruments on laboratory performance according to an embodiment of the present disclosure.
Fig. 9 illustrates a flow chart of the laboratory performance simulation running in parallel to a real laboratory performance to predict future laboratory problems according to an embodiment of the present disclosure.
Fig. 10 illustrates an exemplary user interface dashboard display illustrating how laboratory problems can be identified according to an embodiment of the present disclosure.
Fig. 11 illustrates a graphical method of identifying laboratory problems according to an embodiment of the present disclosure.
Fig. 12 illustrates a flow chart of the laboratory performance simulation running in parallel to a real laboratory performance to predict when reagents will run out according to an embodiment of the present disclosure.
Fig. 13 illustrates an exemplary user interface dashboard display illustrating differences between simulated performances versus current laboratory configuration according to an embodiment of the present disclosure.
Fig. 14 illustrates a flow chart of the laboratory performance simulation running in parallel to a real laboratory performance to predict when test results can be expected or when a test sample may be ready according to an embodiment of the present disclosure.
Fig. 15 illustrates an exemplary user interface dashboard display illustrating forcasting important laboratory events via simulations according to an embodiment of the present disclosure.

### Detailed Description

In the following detailed description of the embodiments, reference is made to the accompanying drawings that form a part hereof, and in which are shown by way of illustration, and not by way of limitation, specific embodiments in which the disclosure may be practiced. It is to be understood that other embodiments may be utilized and that logical, mechanical and electrical changes may be made without departing from the spirit and scope of the present disclosure.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

The use of the 'a' or 'an' can be employed to describe elements and components of the embodiments herein. This is done merely for convenience and to give a general sense of the inventive concepts. This description should be read to include one or at least one and the singular includes the plural unless it is obvious that it is meant otherwise.

The term 'laboratory instrument' or "laboratory device" as used herein can encompass any apparatus or apparatus component operable to execute and/or cause the execution of one or more processing steps /workflow steps on one or more biological samples and/or one or more reagents. The expression 'processing steps' thereby can refer to physically executed processing steps such as centrifugation, aliquotation, sample analysis sample transportation, storage, and the like. The term 'instrument' or 'device' can cover pre-analytical instruments/devices, post-analytical instruments/devices, analytical instruments/devices and laboratory middleware.

The term 'laboratory middleware' as used in the present description can refer to any physical or virtual processing device configurable to control a laboratory instrument/device or system comprising one or more laboratory instruments/devices in a way that workflow(s) and workflow step(s) can be conducted by the laboratory instrument/system. The laboratory middleware may, for example, instruct the laboratory instrument/system to conduct pre-analytical, post analytical and analytical workflow(s)/ workflow step(s) as well as sample transportation step(s). The laboratory middleware may receive information from a data management unit regarding which steps need to be performed with a certain test sample. In some embodiments, the laboratory middleware can be integral with a data management unit, can be comprised by a server computer and/or be part of one laboratory instrument/device or even distributed across multiple instruments/devices of the laboratory automation system. The laboratory middleware may, for instance, be embodied as a programmable logic controller running a computer-readable program provided with instructions to perform operations.

The term "workflow control unit", as used herein can be a broad term and can be given its ordinary and customary meaning to a person of ordinary skill in the art and may not be limited to a special or customized meaning. The term specifically may refer, without limitation, to an electronic device configured, specifically, by hardware and/or by software programming, for controlling the functionality of the sample processing system within the laboratory middleware. The workflow control unit may further be configured for data exchange with the at least one monitoring system and/or at least one cloud server. Specifically, the workflow control unit may be or may comprise a computing device within the laboratory middleware, such as at least one processor, configured for receiving an electronic signal, such as the at least one item of information, from the at least one monitoring system and/or the at least one cloud server, and for further evaluating the received signal. Further, the workflow control unit may be configured for controlling the functionality based on the received and evaluated signal, for example, based on the at least one item of information.

A 'data storage unit' or 'database' can be a computing unit for storing and managing data such as a memory, hard disk or cloud storage. This may involve data relating to biological/medical test sample(s) to be processed by the automated system. The data management unit may be connected to an LIS (laboratory information system) and/or an HIS (hospital information system). The data management unit can be a unit within or co-located with a laboratory instrument/device. It may be part of the laboratory middleware. Alternatively, the database may be a unit remotely located. For instance, it may be embodied in a computer connected via a communication network.

The term 'communication network' as used herein can encompass any type of wireless network, such as a WiFi^{™}, GSM^{™}, UMTS, Bluetooth, Ultra Wideband (UWB), Infrared, Induction, or other wireless digital network or a cable based network, such as Ethernet^{™} or the like. In particular, the communication network can implement the Internet protocol (IP). For example, the communication network can comprise a combination of cable-based and wireless networks.

The term 'remote system' or 'server' as used herein can encompass any physical machine or virtual machine having a physical or virtual processor, capable of receiving; processing and sending data. A server can run on any computer including dedicated computers, which individually can also often be referred to as 'the server' or shared resources such as virtual servers. In many cases, a computer can provide several services and have several servers running. Therefore, the term server may encompass any computerized device that shares a resource with one or more client processes. Furthermore, the terms 'remote system' or 'server' can encompass a data transmission and processing system distributed over a data network (such as a cloud environment).

The term 'simulation' as used herein can encompass the execution of a computer-implemented model to predict a future system behavior (for example status of components, amounts, key performance indicators (KPIs)) based on available data such as, for example, the current status, past data, assumed future data, expected data ranges, and/or probability distributions. Simulation can encompass any use of a model reflecting relevant aspects of the laboratory status and orders, as well as the use of calculations for forecasting any data of interest about a future status or performance of the laboratory expected in the future. A simulation can mimic one or several parts of the real behavior of the laboratory, using a computer implementation of a laboratory model.

A simulation can calculate the status changes of entities moving through the processes (e.g., sample tubes) and the resources needed for the processes (e.g., instruments, human resources, consumables, reagents, and the like) over time. Status changes of the entities can be caused by the processes or activities carried out on the entities such as, for example, entering and exiting a laboratory instrument, waiting before an entity can be processed, the processing time, the transportation of an entity and the like but can also be influenced by the status of the resources (e.g., the laboratory instrument is turned off) and other entities (e.g., occupying the same resources, using the same processes, and the like).

A simulation can be a discrete event simulation, in which the status changes are called "events" or any other method from predictive analytics as machine learning, extrapolation, statistical simulations. In one embodiment, simulation results are used to compute aggregated information about the laboratory such as, for example, the expected future workload of laboratory instruments, transport system, and human resources, occupation of buffers, performance parameters, potential performance, violating issues, resource consumption, costs, or derived events, and the like.

The term 'optimization' as used herein can encompass the activity of identifying the best option (e.g., the schedule of activities, the configuration of laboratory equipment, the configuration of software, the amount of resources, the workflows to follow and resources to use, the location of resources) among several alternatives while considering the constraints (e.g., availability of resources, location of entities) such that one or more objective functions are maximized or minimized. An objective function is a mathematical function, which expresses by numbers how well an option fulfills the quality criteria (e.g., of the laboratory owner/operator, of the medical personnel that ordered the tests, and/or of the patient). An objective function is constructed out of relevant performance indicators such as, for example, turn-around times, time to result, throughput, (in)efficiencies, costs, robustness, workload, and the like. The result of an optimization can comprise one or several options that are good, optimal, or better than the current state, measured by the objective function under consideration of the fulfillment of the constraints. Optimization also includes the actions needed for the implementation of the identified best option in order to achieve the improved objectives. Such actions can encompass making manual adjustments by the laboratory operator with suggestions by the laboratory system and/or computer-based and fully-automated adjustments. All adjustments can share the goal of improving one or more or a weighted combination of the laboratory's key performance indicators (KPIs).

The difference between simulation and optimization is that with optimization a proposal is produced to achieve the goal, like high performance, in the best way. Accordingly, optimization is a prescriptive method rather than a predictive (simulation) method. Methods of the prescriptive analytics can be used for generating laboratory operator suggestions or automatic adjustments. For example, the optimization can propose a new assignment of reagent cassettes to laboratory analyzers in order to achieve a better (optimal) performance, whereas a simulation will be used thereafter for assessing the performance that the newly proposed configuration may achieve. Examples of prescriptive analytics methods are presented in paper P. FESTA, "A brief introduction to exact, approximation, and heuristic algorithms for solving hard combinatorial optimization problems", 16th International Conference on Transparent Optical Networks (ICTON), 2014, pages 1 to 20, doi: 10.1109/ICTON.2014.6876285.

The optimization problem can be solved by an exact optimization method, an approximation of the original problem by a simpler one and solving the simpler problem, and/or heuristics and/or metaheuristics. The exact optimization method can be a (i) branch and bound method, (ii) a dynamic programming method, or (iii) a solver. However, other exact optimization methods can be envisioned and used.

The solver may comprise multiple algorithms (i.e., not only exact ones). The exact optimization method can also comprise combinations of aforementioned methods. Solving the approximated simpler problem may comprise applying (i) one or more greedy algorithms, (ii) a local search, (iii) one or more relaxation-based algorithms, or (iv) one or more random algorithms. Other methods for solving the approximated simpler problem can be envisioned and may be used. Solving the approximated simpler problem may also comprise combinations of aforementioned methods. Heuristics and/or metaheuristics may be (i) a simulated annealing, (ii) one or more evolutionary algorithms, (iii) a tabu search, or (iv) one or more Greedy Randomized Adaptive Search Procedures (GRASP). The heuristics and/or metaheuristics may also comprise combinations of aforementioned methods.

Normally, typical laboratory performance dashboards display current and past laboratory performance. In the laboratory performance dashboard disclosed herein, future laboratory performance can be predicted or forecasted. By running laboratory simulations of the entire laboratory in real time in the background, the following functionalities can become part of the laboratory performance dashboard system:
- The laboratory operator can "look into the future" to get a prognosis. For example, the laboratory operator could obtain times when certain test results will be ready. This can be important for some types of test samples such as, for example, urgent samples, such as, for example, short turn-around-time (STAT) samples for emergency cases. Additionally, the laboratory operator could obtain information about when test samples have completed processing and become available for other processes such as, for example, analyzers to which test samples need to be processed manually or for manual analysis processes. Further, the laboratory operator can be informed when human interactions are needed by the laboratory such as, for example, upcoming laboratory issues, reagent or consumable re-loading or removal of sample racks, predictive maintenance and service activities, waste removal, and the like.
- The laboratory operator can be informed about the consequences of any intended laboratory actions. For example, the laboratory operator can see the effect of changes made to the laboratory configuration such as, for example, changes to test assignments, the number of cassettes, and the like. In addition, in combination with proposals generated by the laboratory forecasting, the laboratory operator could see the effect of choosing one or more proposed laboratory configuration alternatives. Further, the laboratory operator can see the consequences on laboratory performance of a certain choice of sampling loading on, for example, the time for obtaining the test results. Additionally, the laboratory operator can see the consequences on laboratory performance of servicing an analyzer or transportation lines at a certain time of day or the turning certain laboratory systems off or on standby to, for example, save energy. Also, the laboratory operator can see the potential gains in laboratory performance when using aliquoting or, for example, centrifuging some test samples on a separate stand-alone centrifugation system instead of only using a centrifuge connected to the fully-automated laboratory system.

One or more of the following aspects should be considered for the laboratory simulator. Firstly, when analyzing new configurations, the models for the laboratory simulations can get their configuration input from the laboratory optimizer and the order lists from the laboratory middleware. The order lists are processed to, for example, scale-up the order load of the laboratory system. Alternatively, the laboratory configuration can be entered/changed by the laboratory operator directly such as, for example, by manually defining where, which, and how many test cassettes should be installed. However, the changes that can be made manually or proposed by the optimizer automatically depend on the situations and laboratory operator wishes. For example, during design time, laboratory hardware can be varied such as constrained by e.g., space limitations. During run-time, when the laboratory systems have been installed, only, for example, reagent cassette placements on the existing laboratory hardware can be optimized. The laboratory configuration designer may add preferences of the customers such as, for example, the preferred type of laboratory hardware or which analyzers should carry identical types of tests and the like.

Secondly, for analyzing the impact of changes in the laboratory configuration or availability of laboratory analyzers such as, for example, during laboratory service or maintenance, the laboratory operator can define laboratory events such as, for example, when to turn off certain laboratory analyzers or other hardware.

Thirdly, the status of the simulation/configuration can be synchronized with the current status information of the laboratory in order to enhance the predicted laboratory performance. For example, if the laboratory models are not 100% accurate or if other delays occur, test samples may enter the laboratory analyzers earlier or later in real time when compared to the laboratory simulation. A synchronization of the laboratory simulation periodically can help correct these deviations. The information needed for synchronization can be obtained from known laboratory event data from the laboratory middleware, which can also include when and which laboratory analyzers are masked/unmasked.

Fourthly, laboratory configuration changes can be synchronized with data from the laboratory middleware and/or the laboratory analyzers such as, for example, which analyzers are masked, the placement of reagent packs, and/or the sample scheduling configurations of the laboratory middleware.

Fifthly, the laboratory operator or the laboratory system itself can trigger a process in which order data such as, for example, from multiple days, from the laboratory middleware is used to feed an optimizer. One or multiple proposals for possible laboratory configurations can be obtained and simulated. Optionally, the laboratory operator can select a scenario from the different proposals to be simulated. The dashboard can then show the differences in laboratory performances values for the current laboratory configuration versus alternative laboratory configurations. For example, the laboratory operator can observe the impact of changing the laboratory configuration of test assignments to laboratory analyzers on the turn-around-times (TATs) or walk-way times, impact of the human resource scheduling, including pause times on the TATs or working times such as when is the last test sample processed, the impact of turning off or servicing laboratory instruments at certain times on the TATs, and the like.

Sixthly, a dashboard that shows one or more laboratory performance indicators of past, current, or future time as well as a comparison with the same laboratory indicators of the current laboratory system, based on real data such as:
- Simulated throughputs
- Simulated TATs of test samples such as, for example, TAT from entering the laboratory until the test results were published, TAT from the order being entered by the physician until the test results are sent to the physician, the TAT of the pre-analytics systems, and/or the TAT of the serum-work-area analysis.
- Simulated times to result
- Simulated buffer levels
- Simulated test sample traffic intensities
- Simulated laboratory instrument loads and workload of human resources
- Simulated idle times
- Simulated number of test samples or tests such as, for example, absolutely or relatively to the total number, exceeding the laboratory performance acceptance criteria such as, for example, percentage of test samples or test results inside or outside the defined maximum time to result criterion
- Simulated point-to-point traveling time
- Simulated waiting times, non-productive times such as, for example, in buffers
- Simulated walk-away times or number of required human interactions per time
- Indicators of costs or benefits of alternative laboratory configurations compared with the current laboratory configuration such as, for example, numbers of quality controls (QCs) per day, costs for QCs per day, wasted number of tests and/or costs due to reagent expiration

Seventhly, a dashboard that shows the laboratory operators, laboratory system owners and/or laboratory service/maintenance providers information about the predicted upcoming laboratory activities and events with an indication of when these events are expected such as, for example, in the next 10 minutes, 30 minutes, one hour, half day, per week, shift, day or week, such that the laboratory operator can anticipate these events. Examples of these events and activities are:
- An estimation of when reagents, consumables, and/or disposables will run out and a refill will be needed. A level of, for example, reagents may be low but if they are not ordered frequently, the walk-away time may be longer than if they are ordered frequently.
- An estimation of the duration until a small or larger laboratory service/maintenance activity will be needed, to be carried out by the system operator or a technician may be needed. This information can be shown on the laboratory system only or can be automatically communicated to a laboratory service provider such as, for example, customer service, so that a service visit can be scheduled

Eighthly, the laboratory simulations used to determine operation confidence intervals by, for example, performing many laboratory simulations with the same partial stochastic parameters such as, for example, order distributions, variation in processing times, and the like. These confidence intervals can be used to compare with the real laboratory system. If the real laboratory system deviates too much negatively from the simulation-determined intervals, this is an indication that the real laboratory system needs improvement. By laboratory simulation, the potential origin of the deviation can be determined as well as the sensitivity to the potentially causing parameters of the deviations such as, for example, delayed laboratory operator interaction.

In addition, a laboratory simulation can include an optimization-based scheduler to simulate an ideal working laboratory system where, for example, the manual interactions can take place at the right times. The simulation will indicate the laboratory performance that can be achieved versus the real laboratory performance to indicate to the customer that improvements can be possible with better scheduling. The scheduler in the simulator can indicate how to achieve the optimal laboratory performance.

Additionally, a laboratory simulation can be used to provide missing information from a part of the laboratory system. Examples of missing information are:
- Missing knowledge about the behavior of a third party provider that makes it difficult to include in a well-scheduled laboratory system. With machine learning or statistical data, a model can be created of the third party provider that can be included into the scheduler
- An laboratory instrument that does not communicate the necessary information such as, for example, expected time to test result, load levels for its buffers and resources, and the like, can be simulated in order to obtain the information.

Further, laboratory simulations can be continuously adjusted by real time laboratory events. As laboratory simulation models are usually simplifications of real world events, the forecasts will become less accurate the further into the future it is looked. By continuously adjusting the status of the models according to recent real time laboratory data, the predictions, or forecasts, can be optimally predicted, or forecasted.

Lastly, the differences between reality and the model can also be analyzed to trigger alarms/warnings or to simply inform the laboratory operator that the real laboratory system is behaving differently to that that was predicted. Examples include:
- Trend detection that certain elements show an increasing difference from the ideal state such as, for example, aging of components such that with increasing frequency time differences exist between the real laboratory events versus the modeled laboratory events. These trends can be indicated on the dashboard and, via extrapolation, an estimate can be given for the remaining time before a laboratory service/parts renewal
- Abnormality detection via, for example, machine learning, that can identify unique laboratory events that differ from normal laboratory operation. These can be, for example, delays due to unique laboratory events such as, for example, a short jam of a test sample, a test sample traffic congestion, and a combination of factors that leads to an unacceptable situation, for example, logical processing rules in combination with a certain order list that resulted in a wrong prioritization and, therefore, an unacceptable turn-around-time. The laboratory operator can be informed about these situations, including the time when the problem occurred. In this case, the recorded and simulated laboratory event data can be played back for analysis as well as to help find a solution.

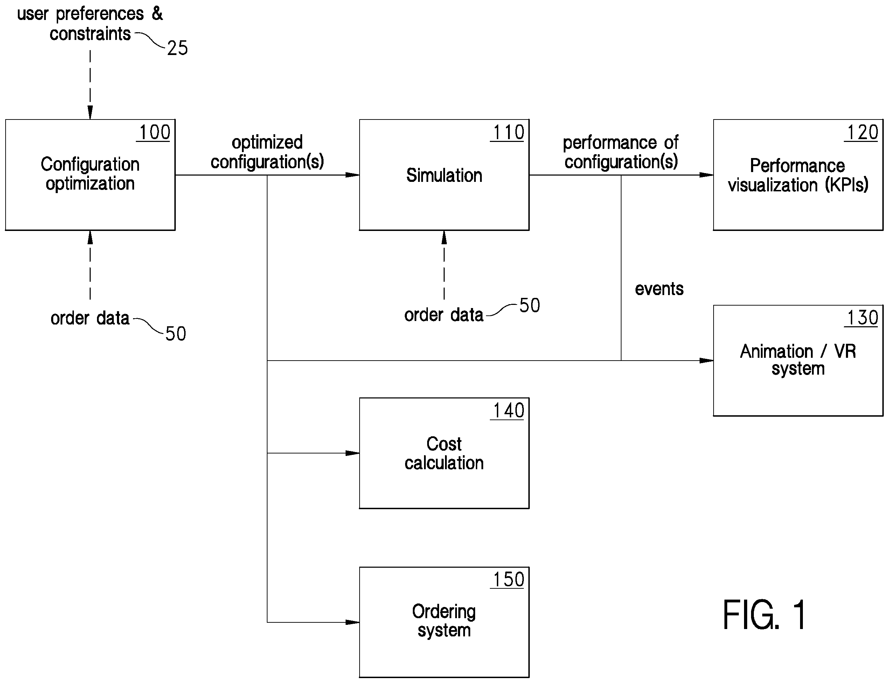

Referring initially to Fig. 1, Fig. 1 illustrates a flow chart of the laboratory configuration optimizer and performance configuration simulation during design phase according to an embodiment of the present disclosure. Initially, the laboratory configuration module 110 determines an optimized laboratory configuration using a laboratory configuration optimizer. Laboratory operator preferences and constraints 25 and laboratory order data 50 are used as inputs into the laboratory configuration optimizer. The resulting optimized laboratory configuration(s) are fed to the simulation module 110 in order to simulate how the resulting optimized laboratory configuration(s) may perform. The laboratory order data 50 is also fed into the simulation module 110 in order to provide input into the simulation laboratory. From the simulation module 110, the output or performance of the simulated laboratory configuration is sent to a performance visualization module 120 or dashboard for the laboratory operator to see how the resulting optimized laboratory configuration(s) performs.

In addition, the resulting optimized laboratory configuration(s) are then used by the ordering system module 150 to facilitate the ordering of supplies for the laboratory. Additionally, the resulting optimized laboratory configuration(s) can be used by the cost calculation module 140 to facilitate the calculation of costs associated with the laboratory.

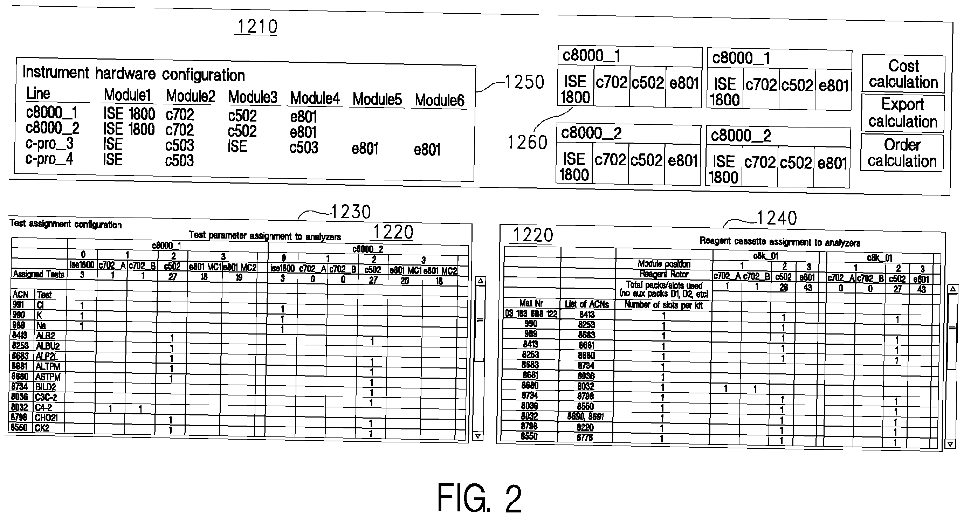

Fig. 2 provides an exemplary user interface dashboard display during the laboratory design phase. The optimized laboratory hardware is displayed on the top half 1210 of the dashboard and the test assignment configurations are displayed on the lower half 1220 of the dashboard. The optimized laboratory hardware 1210, i.e., analyzers, is displayed linearly on the top left 1250 and as figure modules on the top right 1260 of the dashboard. The test assignments 1220 may comprise on the lower left of the dashboard a list of parameters 1230 assigned to each of the laboratory modules along with their submodules such as, for example measurement work cell or reagent rotor. Since reagent cassettes may be used for multiple test parameters, a list with cassette material numbers can be listed along with physical position of the reagent cassette on the lower right 1240 of the dashboard display.

Optionally, the resulting optimized laboratory configuration(s) can also be fed into an animation/virtual reality (VR) module 130 in order to provide a visual representation of the resulting optimized laboratory configuration(s) to the laboratory customers or other laboratory personnel.

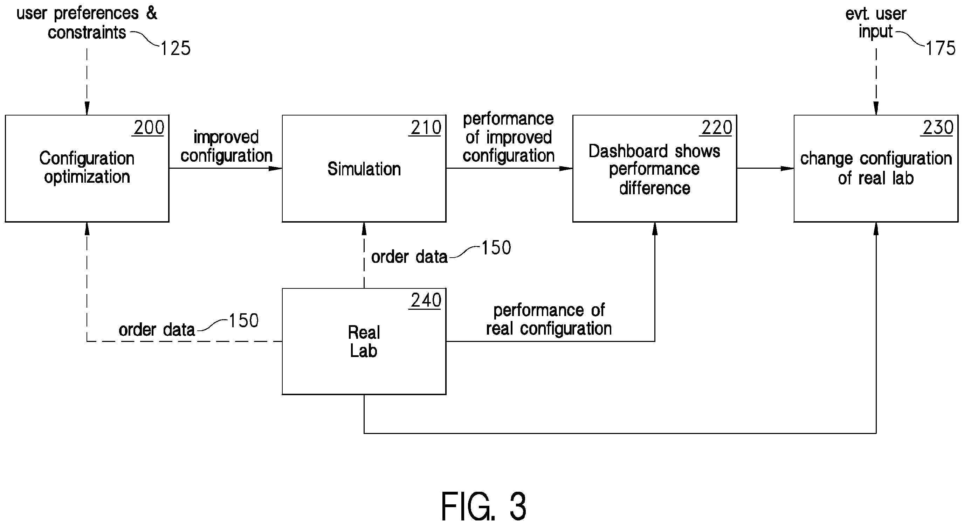

Turning to Fig. 3, Fig.3 illustrates a flow chart of the run-time application of laboratory configuration with simulation according to an embodiment of the present disclosure. In Fig. 3, the laboratory configuration optimization module 200 proposes, based on order data 150 from the actual laboratory 240 and laboratory operator preferences and constraints input 125, the laboratory configuration to be simulated. The proposed optimized laboratory configuration is then inputted into the laboratory performance simulation module 210. The order data 150 from the actual laboratory 240 is also inputted into the laboratory performance simulation module 210. The simulated laboratory performance from the laboratory performance simulation module 210 and the actual laboratory performances from the actual laboratory 240 will be compared and displayed on a dashboard 220 for the laboratory operator. The laboratory operator can then select 230 which proposed laboratory configuration displayed on the dashboard 220 along with other laboratory operator input 175 to reconfigure if at all the actual laboratory 240.

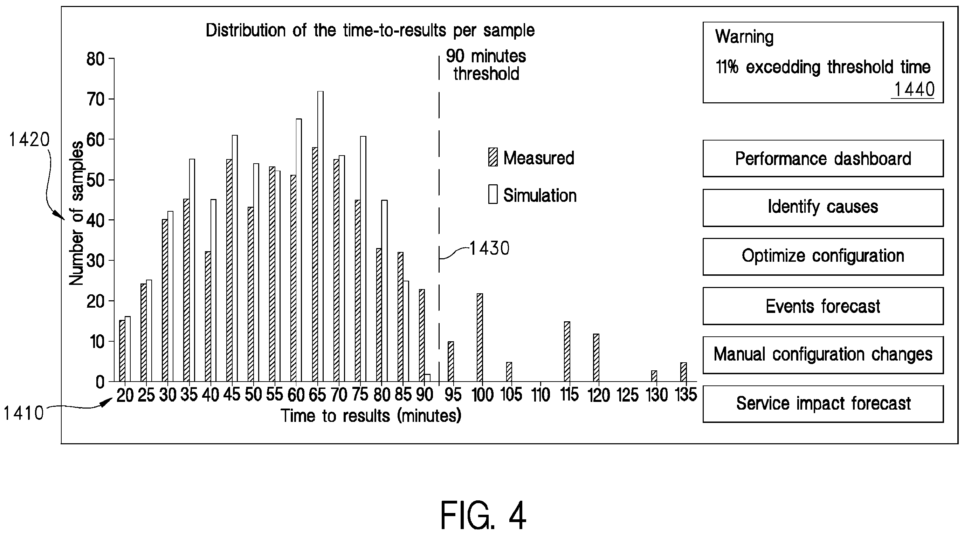

Fig. 4 illustrates an exemplary actual laboratory performance dashboard display illustrating a histogram of time to results, i.e., the distribution of the time-to-results per test sample. The x-axis 1410 indicates the TAT times. The y-axis 1420 indicates the number of test samples with that particular TAT. The dashed line 1430 indicates a target cutoff, or time threshold, i.e., the time at which all test sample results should be available. In this example, the target is to have all test sample results within 90 minutes. For the test sample results that exceed this target, a warning box 1440 is displayed on the dashboard. In this example, 11% of the test sample results exceeded the 90-minute target time. The simulated laboratory performance, however, could have indicated that it would have been possible to achieve the 90-minute target time for test sample results.

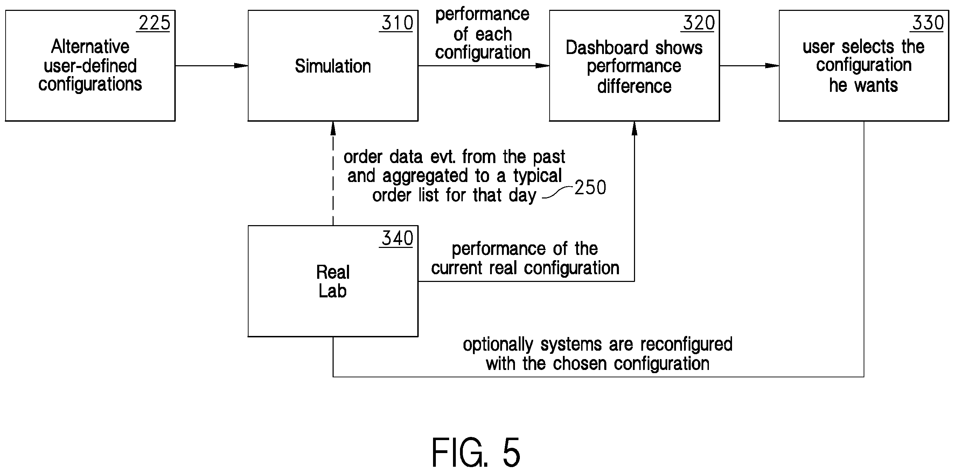

Fig. 5 illustrates a flow chart of the run-time application of laboratory configuration with simulation with operator input according to an embodiment of the present disclosure. Fig. 5 is similar to Fig. 3 except in this embodiment, instead of having a laboratory configuration optimization module 200, the laboratory operator proposes the changes to the laboratory configuration 225 which are then inputted into the laboratory performance simulation module 310 along with order data 250 from the actual laboratory 240 and the laboratory performance of the inputted laboratory configuration is simulated. In one embodiment, the order data 250 from the actual laboratory 240 can be historical order data and aggregated to a typical order list for that day. The simulated laboratory performance from the laboratory performance simulation module 310 and the actual laboratory performances from the actual laboratory 340 will be compared and displayed on a dashboard 320 for the laboratory operator. The laboratory operator can then choose 330 which laboratory configuration can then be implemented. The laboratory operator may select simulated laboratory configuration if the simulated laboratory performance is better than the actual laboratory performance.

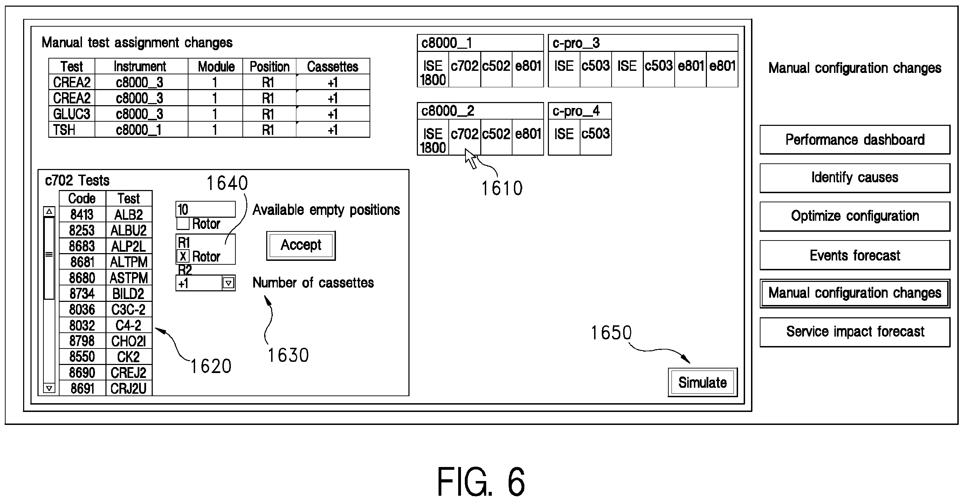

Fig. 6 illustrates an exemplary user interface dashboard display illustrating a laboratory operator manually changing the laboratory configuration. A laboratory operator can select a laboratory analyzer module 1610 of the displayed laboratory analyzer modules in the upper right corner of the dashboard. After selecting the laboratory analyzer module 1610, a list of installed and possible test parameters 1620 for that laboratory analyzer module 1610 is displayed. These test parameters can be selected by the laboratory operator and assigned to a laboratory submodule 1640 such as, for example, a reagent rotor. By selecting the number of reagent cassettes to be added (+1) or removed (-1) 1620, the test assignment can be changed by the laboratory operator. If the laboratory operator selects the simulate button 1650, the laboratory performance of new laboratory configuration can be simulated before actually implementing the laboratory configuration in the laboratory in order to see if this new laboratory configuration performs better than the current laboratory configuration.

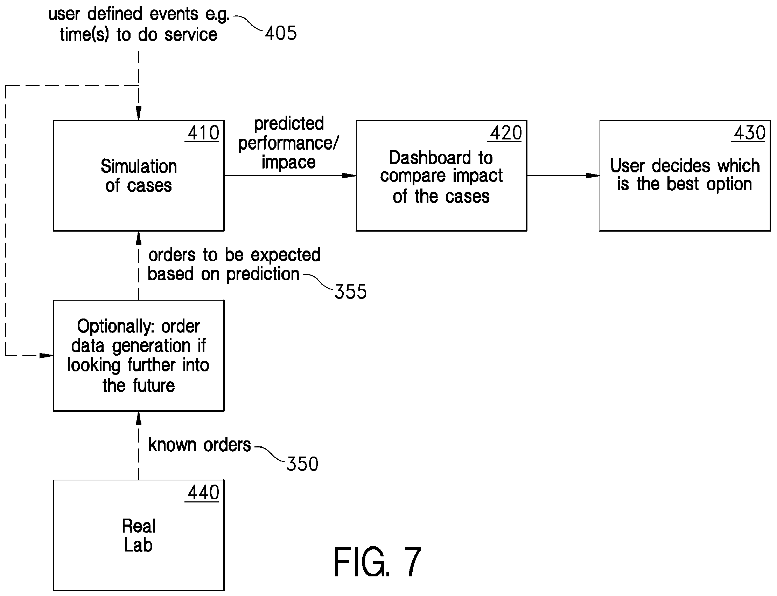

Fig. 7 illustrates a flow chart of the use of a laboratory performance simulation module 410 to propose times for performing a laboratory service with the least amount of disruption to laboratory performance according to an embodiment of the present disclosure. In this embodiment, the laboratory performance simulation module 410 can propose a time for conducting laboratory maintenance or service on a laboratory instrument such as, for example, a time if a one of the laboratory analyzers is offline, it will not result in too much negative impact on laboratory performance. Different cases/times can be proposed by the laboratory operator 405, i.e., different times at which to start the laboratory service/maintenance and/or which laboratory instruments to service, as input into the laboratory performance simulation module 410 in order to see the impact on overall laboratory performance. Known order data 350 from the actual laboratory 440 can also be inputted into the laboratory performance simulation module 410. In addition, in one embodiment, predicted order data 355 based on predicted/forecasted order data can also be inputted into the laboratory performance simulation module 410. The simulated laboratory performance from the laboratory performance simulation module 410 can then be displayed on a dashboard 420 for the laboratory operator. The laboratory operator can then select 430 the best times to schedule laboratory service/maintenance and on which laboratory instruments based on simulated impact of the laboratory service on laboratory performance.

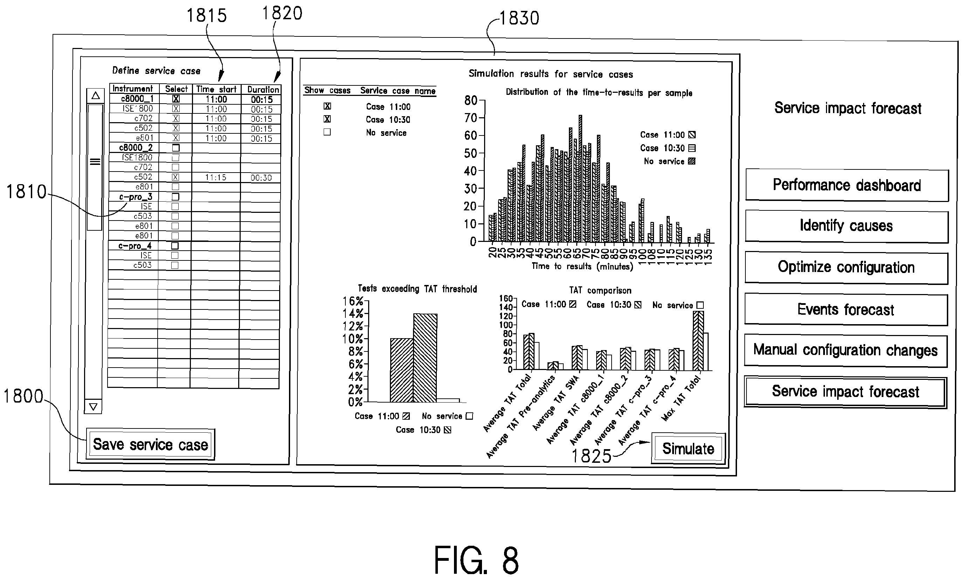

Fig. 8 illustrates an exemplary user interface dashboard display illustrating the impact of servicing instruments has on laboratory performance. On the left side of the dashboard display 1800, laboratory instrument lines or individual laboratory modules 1810 are displayed and can be selected for servicing. The start time 1815 and duration of the laboratory service 1820 can also be selected. Several laboratory service scenarios, or cases, can be selected at the same time. The different laboratory service cases can be simulated by the laboratory operator selecting the simulate button 1825. The results of the laboratory simulation can be displayed and compared in the simulation display box 1830. In one embodiment, the scenario of no laboratory service can also be displayed for comparison. Thus, the laboratory operator can determine the impact of laboratory service on laboratory performance and can choose the scenario with the least amount of impact on laboratory performance such as, for example, the scenario with the least amount of impact on the targeted TAT time. The laboratory operator can then choose the optimal time, i.e., the time of least impact on laboratory performance, to have service be performed on the laboratory instrument.

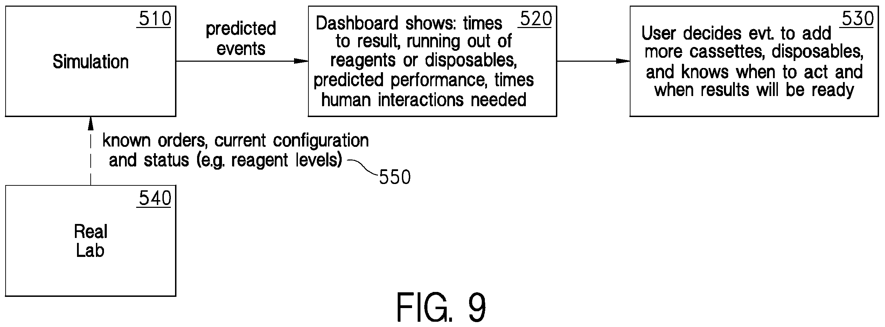

Fig. 9 illustrates a flow chart of the simulation laboratory performance running in parallel to a real laboratory performance to predict future problems such as, for example, running out of reagents and/or other consumables according to an embodiment of the present disclosure. The times at which these unwanted events could occur can be shown on the dashboard. In this embodiment, the known order data, current laboratory configuration, and consumable status (i.e., for example, reagent levels) 550 from the actual laboratory 540 can be inputted into the laboratory performance simulation module 510. The predicted laboratory performance events from the laboratory performance simulation module 510 are then displayed on a dashboard 220 for the laboratory operator. The predicted laboratory performance events can comprise, for example, predicted laboratory test result times, predicted times that the laboratory may run out of reagents or other consumables, predicted laboratory performance, and/or predicted times that a laboratory operator may need to intervene with the laboratory system such as, for example, for laboratory service or maintenance. The laboratory operator can then decide 530 when to refill reagent cassettes or other consumables as well having an estimate of when the laboratory operator's action may be needed and/or when laboratory test results may be ready.

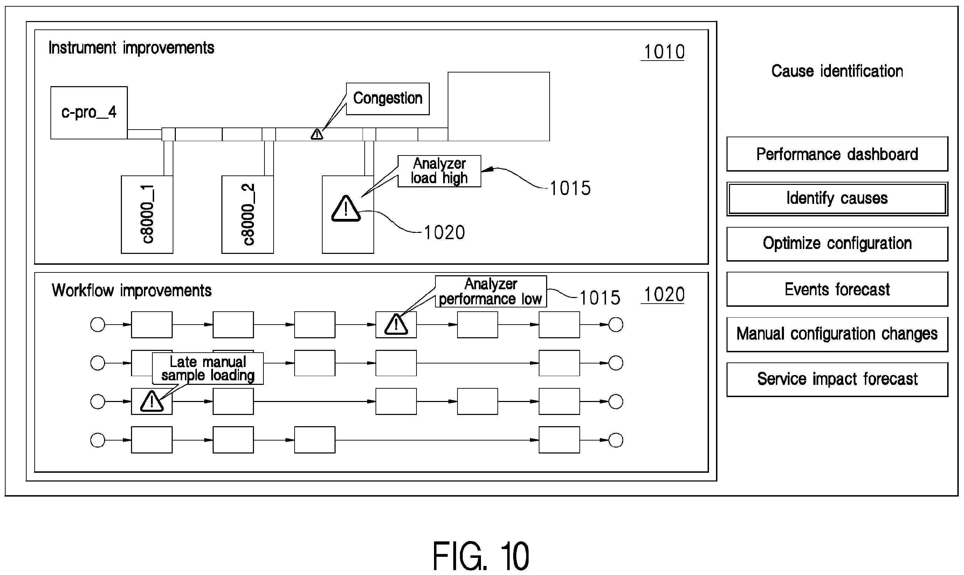

Fig. 10 illustrates an exemplary user interface dashboard display illustrating how laboratory problems can be identified. By comparing the simulation, i.e., the ideal laboratory performance, and the actual laboratory performance, laboratory bottlenecks 1020 (indicated as triangle warning icons) can be identified. The bottlenecks 1020 can be shown by indicating, for example, in which laboratory module the delays are occurring (the top part of the dashboard 1010) or by indicating the workflow steps in which the steps were not performing optimally (the bottom part of the dashboard 1020). By selecting the bottleneck icon 1020, or by hovering a cursor over the bottleneck icon 102, a text window 1015 can appear that displays additional details about the type of problem occurring at that spot.

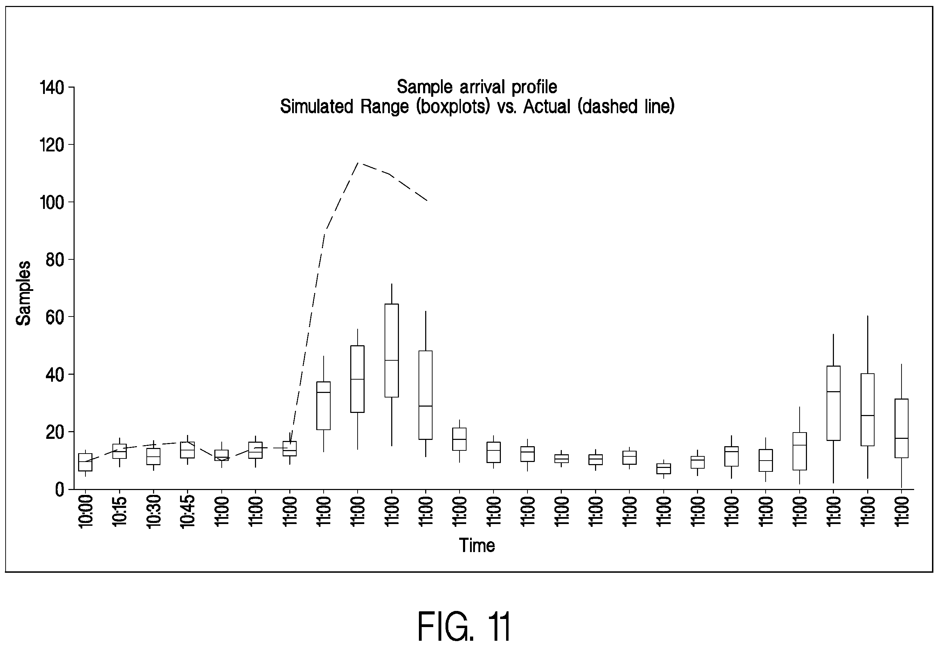

Fig. 11 illustrates a graphical method of another way of identifying laboratory problems. In this embodiment, the assumption of the simulation (e.g., the test sample arrival profile graphed as boxplots) can be compared to the actual data (i.e., dashed line) of the test sample arrival profile. Based on the results of this type of comparison, new simulations/optimizations can be triggered.

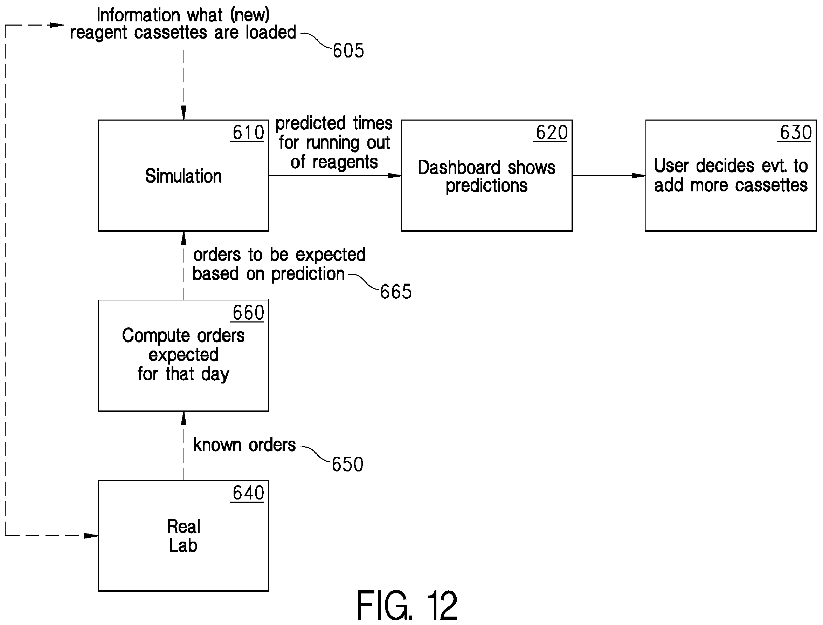

Fig. 12 illustrates a flow chart of the simulation laboratory performance running in parallel to a real laboratory performance to predict when reagents will run out according to an embodiment of the present disclosure. In this example, the information of when reagent cassettes are loaded may be automatically 605 provided by the laboratory system and inputted into the laboratory performance simulation module 610. In this embodiment, the known order data 650 from the actual laboratory 640 are inputted into the laboratory performance simulation module 610. The predicted time that the laboratory may run out of reagents simulated by from the laboratory performance simulation module 610 are then displayed on a dashboard 620 for the laboratory operator. The laboratory operator can then decide 630 when to refill or replace the reagent cassettes.

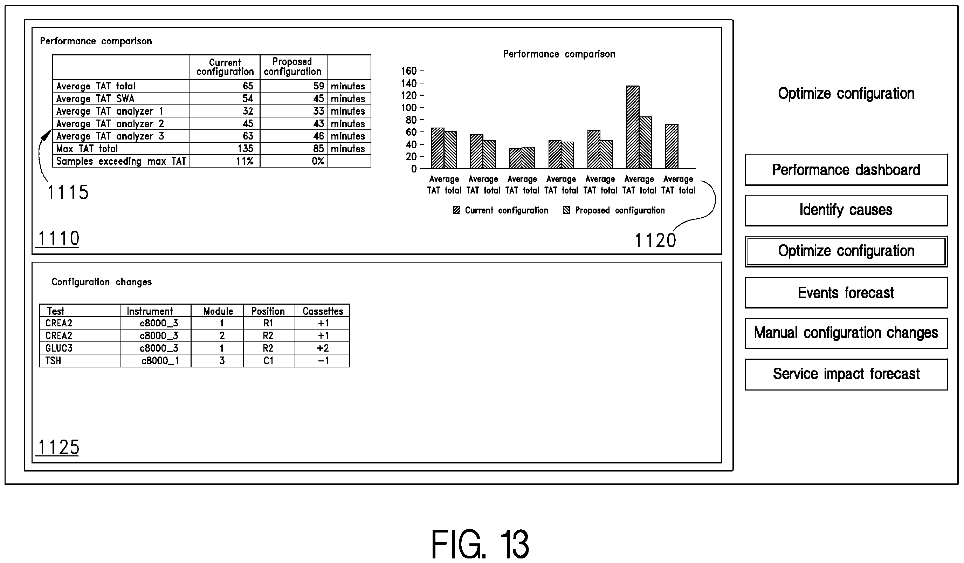

Fig. 13 illustrates an exemplary user interface dashboard display illustrating differences between simulated performances versus current laboratory configuration. If a laboratory operator wishes to optimize a laboratory configuration because, for example, the current laboratory configuration is not currently optimize for the current or planned test orders, the dashboard can display to the laboratory operator the differences between, for example, the simulated laboratory performance of the optimized laboratory versus the current laboratory design without optimization in the upper portion of the dashboard 1110. In this section of the dashboard 1110, laboratory performance indicators can be shown as numbers 1115 such as, for example, TATs, as well as graphical comparisons 1120. The bottom section of the dashboard 1125 displays the proposed laboratory changes such as, for example, changing the reagent cassette configuration of the laboratory analyzers. In one embodiment, the dashboard provides only information. However, in another embodiment, the proposed laboratory configurations can be applied to the current laboratory configuration.

Additionally, Fig. 12 also illustrates a way to look further into the future. If not all orders are known yet or the arrival times of test samples from those orders are not known resulting in the laboratory running out reagents. The laboratory performance simulation module 610 may use predicted order information based on the current laboratory situation and information provided from the past such as, for example, similar days. The predicted number of orders expected that day can therefore be calculated 660 and inputted 665 into the laboratory performance simulation module 610.

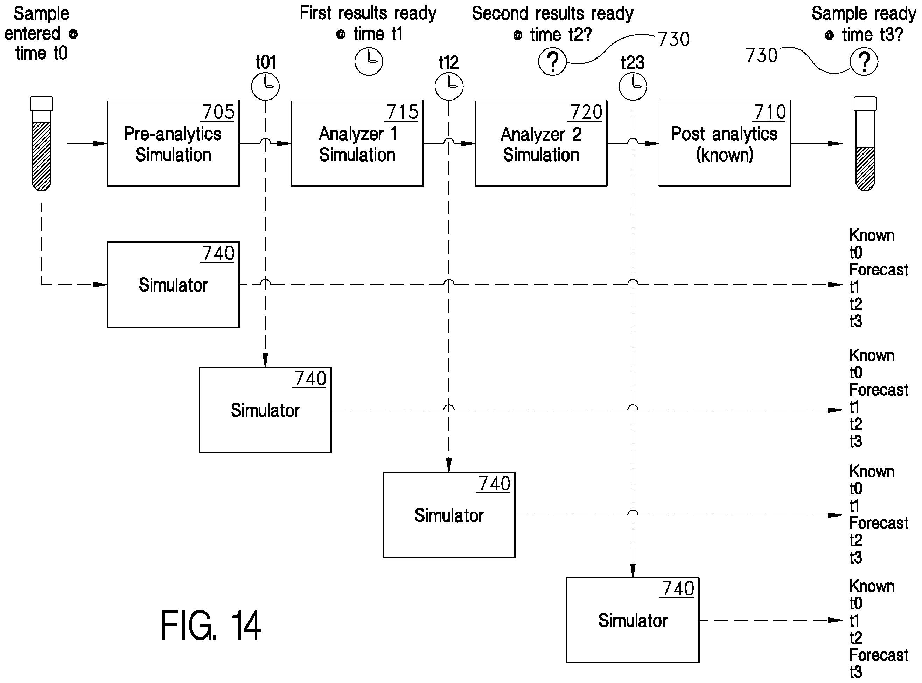

Fig. 14 illustrates a flow chart of the laboratory performance simulation running in parallel to a real laboratory performance to predict when test results can be expected or when a test sample may be ready according to an embodiment of the present disclosure. In this figure, the pre-analytic 705 and post-analytic devices 710 are known to the laboratory system, i.e., there are event models available for these devices. Laboratory analyzer 715 is also known but laboratory analyzer 720 is not known, i.e., there is no event model available for laboratory analyzer 720. Unknown elements can lead to unknown times, illustrated by a question mark 730 in Fig. 13. Since all time events of a real laboratory system are communicated, this unknown device element can be replaced with a simulation machine learning model that will become better over time as it receives more input. The laboratory performance simulation model can then make predictions/forecasts for the events t1...t3. The predictions/events can be displayed and updated on a laboratory dashboard.

Since the laboratory performance simulation model is not perfect, the times provided by the laboratory performance simulation module 740 to the laboratory dashboard can be updated by the real times as they occur, i.e., in Fig.13, when t01 (the time the sample leaves the pre-analytic device 705), tl2 (the time the sample leaves the laboratory analyzer 715), and t23 (the time the sample leaves the laboratory analyzer 720) occur. As real time results are received and added to the laboratory performance simulation module 740, a better prediction/forecast for the remaining event times can result.

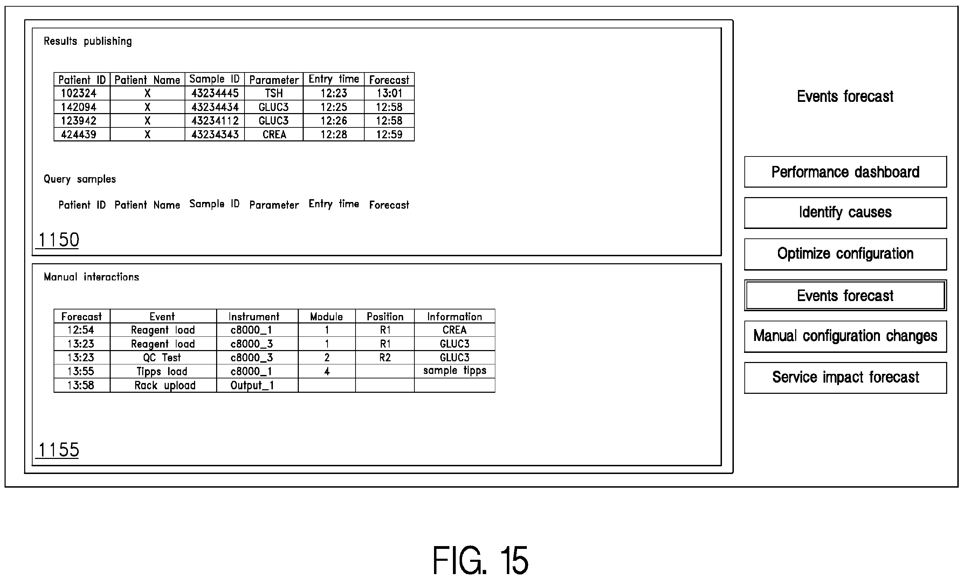

Fig. 15 illustrates an exemplary user interface dashboard display illustrating forcasting important laboratory events via simulations. In the upper part of the dashboard display 1150, the laboratory operator can observe when the test sample results of certain test sample selected by the laboratory operator will be published, i.e., forecasted. The lower section of the dashboard display 1155 displays events that require manual intervention by the laboratory operator. This section of the dashboard displays to the laboratory operator when, for example, reagents may run out or when an output buffer may become full and the test sample racks need to be unloaded from the laboratory system.

The technologies providing the predictive information can comprise an integration of event simulation with dashboard and/or control software. Furthermore, the models for the event simulation can be mathematical, heuristic, statistical or logical deterministic or partial stochastic formulations as known in the art. Models inside the simulations can include a learning heuristic that can be trained in real time by real event data to obtain an increasingly realistic behaviour over time. Different simulations of same laboratory systems, or parts of the laboratory system, may be run in parallel using different types of models, which can then be compared and the best predicting simulation model can then be chosen for delivering the results for the laboratory performance dashboard. For example, for learning simulation models, which tend to perform poorly initially but perform better over longer training time, non-learning simulation models will initially provide the information to the laboratory performance dashboard. The laboratory system can identify how well each simulation model is performing by comparing the forecasted events with real laboratory data. Depending on which simulation model performs best, the laboratory system will decide which results to include as information or how to weigh the results to distill a prediction or forecast. Additionally, the simulation models inside the simulations can also include statistical descriptions such as, for example, distributions.

For the event simulations, software code will be written and/or existing simulation software/libraries will be incorporates. Examples of such existing simulation software/libraries can be Simio^{™}, AnyLogic^{™}, Arena^{™}, Plant Simulation^{™}, Python with Simpy^{™} libraries, and the like.

Further disclosed and proposed is a computer program product including computer-executable instructions for performing the disclosed method in one or more of the embodiments enclosed herein when the program is executed on a computer or computer network. Specifically, the computer program may be stored on a computer-readable data carrier or a server computer. Thus, specifically, one, more than one or even all of method steps as indicated above may be performed by using a computer or a computer network, preferably by using a computer program.

As used herein, a computer program product refers to the program as a tradable product. The product may generally exist in any format, such as in a paper format, or on a computer-readable data carrier on premise or located at a remote location. Specifically, the computer program product may be distributed over a data network (such as a cloud environment). Furthermore, not only the computer program product, but also the execution hardware may be located on premise or in a cloud environment.

Further disclosed and proposed is a computer-readable medium comprising instructions which, when executed by a computer system, cause a laboratory automation system to perform the method according to one or more of the embodiments disclosed herein.

Further disclosed and proposed is a modulated data signal comprising instructions, which, when executed by a computer system, cause a laboratory automation system to perform the method according to one or more of the embodiments disclosed herein.

Referring to the computer-implemented aspects of the disclosed method, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

It is noted that terms like "preferably," "commonly," and "typically" are not utilized herein to limit the scope of the claimed embodiments or to imply that certain features are critical, essential, or even important to the structure or function of the claimed embodiments. Rather, these terms are merely intended to highlight alternative or additional features that may or may not be utilized in a particular embodiment of the present disclosure.

Having described the present disclosure in detail and by reference to specific embodiments thereof, it will be apparent that modifications and variations are possible without departing from the scope of the disclosure defined in the appended claims. More specifically, although some aspects of the present disclosure are identified herein as preferred or particularly advantageous, it is contemplated that the present disclosure is not necessarily limited to these preferred aspects of the disclosure.

## Claims

1. A computer-implemented method of forecasting future laboratory performance of a laboratory system comprising a plurality of laboratory instruments configured to perform tests on laboratory test samples, a laboratory middleware, a control unit, and a dashboard display communicatively connected via a network communication connection, the method comprising:
providing laboratory operator preferences and laboratory constraints from a laboratory operator to an optimization module of the control unit;
providing laboratory input data and order data to the optimization module of the control unit;
optimizing laboratory configuration based on the laboratory operator preferences, laboratory constraints, laboratory inputs, and order data at the optimization module of the control unit;
simulating future laboratory performance of the laboratory system by a simulation module of the control unit based on the optimized laboratory configuration provided by the optimization module and real-time laboratory inputs and order data; and
displaying the simulated future laboratory performance and actual laboratory performance on the dashboard display to the laboratory operator.

2. The computer-implemented method according to claim 1, wherein the laboratory input data and order data is continuously provided by the laboratory middleware in real-time, wherein the real-time laboratory data comprises timing for masking of analyzers, reagent pack assignments and placements, sample loading scheduling, and/or combinations thereof.

3. The computer-implemented method according to claims 1 and 2, wherein the simulated future laboratory performance comprises predicting arrival of test results from the plurality of laboratory instruments.

4. The computer-implemented method according to any of the preceding claims, wherein the displayed simulated future performance comprises simulated throughputs, TAT of samples, times to results, buffer levels, sample traffic intensities, instrument loads and workload of laboratory operators, idle times, number of samples, reagents, or tests exceeding a performance acceptance criteria, point-to-point travel times, buffer wait times, walk-away times, number of laboratory operator interactions per time, number of laboratory operators needed, power consumption, water consumption, operational costs, or combinations thereof.

5. The computer-implemented method according to any of the preceding claims, wherein the displayed simulated future performance comprises indicators of benefits of simulated laboratory configuration compared to current laboratory configuration.

6. The computer-implemented method according to any of the preceding claims, further comprising,
changing laboratory configuration of current laboratory via input from the laboratory operator based on the simulated future laboratory performance.

7. The computer-implemented method according to any of the preceding claims, further comprising,
triggering an alert when the simulated future laboratory performance and actual laboratory performance deviate from an acceptable level and/or simulated test samples arrivals and actual test samples arrivals deviate from an acceptable level and/or an abnormality is detected; and
indicating on the dashboard display a potential source of the deviation or abnormality.

8. The computer-implemented method according to any of the preceding claims, further comprising,
calculating estimated future orders based on the optimized laboratory configuration.

9. The computer-implemented method according to any of the preceding claims, further comprising,
calculating sample loading effects on the simulated future laboratory performance.

10. The computer-implemented method according to any of the preceding claims, further comprising,
scheduling manual interactions with the laboratory system based on the optimized laboratory configuration.

11. The computer-implemented method according to any of the preceding claims, further comprising,
displaying predicted upcoming events with an indication when these predicted upcoming events are expected to occur, wherein the predicted upcoming events are estimations of when refills are needed and/or estimations of the start time of maintenance events and/or estimations of the duration of maintenance events and/or predicting future laboratory maintenance events.

12. A computer-implemented method of forecasting future laboratory performance of a laboratory system comprising a plurality of laboratory instruments configured to perform tests on laboratory test samples, a laboratory middleware, a control unit, and a dashboard display communicatively connected via a network communication connection, the method comprising:
providing different configurations of the laboratory system from a laboratory operator to a simulation module of the control unit;
continuously providing real-time laboratory input data from the plurality of laboratory instruments to the laboratory middleware;
continuously providing the real-time laboratory input data and order data from the laboratory middleware to the simulation module;
simulating future laboratory performance of the different configurations of the laboratory system by a simulation module of the control unit based on the real-time laboratory inputs and order data; and
displaying the simulated future laboratory performances of the different configurations of the laboratory system and actual laboratory performance on the dashboard display to the laboratory operator.

13. The computer-implemented method according to claim 12, further comprising,
selecting by the laboratory operator one of the different configurations of the laboratory system based on the simulated future laboratory performances of the different configurations; and
reconfiguring the laboratory system based on the selected configuration.

14. The computer-implemented method according to claims 12 and 13, further comprising,
optimizing future laboratory performances of the different configurations based on laboratory operator preferences, laboratory constraints, real-time laboratory inputs, and order data at an optimization module.

15. A laboratory system for forecasting future laboratory performance, the laboratory system comprising:
a plurality of laboratory instruments configured to perform tests on laboratory test samples;
a laboratory middleware communicatively connected to the plurality of laboratory instruments;
a dashboard display configured to display performance information of the laboratory system; and
a control unit comprising an optimization module and a simulation module and connected to the plurality of laboratory instruments and the laboratory middleware via the network communication connection, the control unit configured to
provide laboratory operator preferences and laboratory constraints from a laboratory operator to the optimization module,
continuously provide real-time laboratory input data from the plurality of laboratory instruments to the laboratory middleware,
continuously provide the real-time laboratory input data and order data from the laboratory middleware to the optimization module,
optimize laboratory configuration based on the laboratory operator preferences, laboratory constraints, real-time laboratory inputs, and order data at the optimization module,
simulate future laboratory performance of the laboratory system by the simulation module based on the optimized laboratory configuration provided by the optimization module and real-time laboratory inputs and order data, and
display the simulated future laboratory performance and actual laboratory performance on the dashboard display to the laboratory operator.
